# EUROPEAN PATENT APPLICATION

(11) **EP 4 306 512 A1**
(43) Date of publication of application: **17.01.2024**
(21) Application number: 22382680.1
(22) Date of filing: 15.07.2022
(51) Int. Cl.: C07D 215/18, C07D 215/20, C07D 215/36, A61K 31/46, A61K 38/49, A61K 45/06, A61P 9/10

(54) **QUINOLYLNITRONES WITH IMPROVED POTENCY**

(71) Applicant: Fundación para la Investigación Biomédica del Hospital Universitario Ramón y Cajal, 28034 Madrid (ES); Consejo Superior de Investigaciones Científicas (CSIC), 28006 Madrid (ES); Universidad Camilo José Cela (Centro de Enseñanza Universitaria SEK, S.A.), 28692 Villanueva de la Cañada, Madrid (ES)
(72) Inventor: ALCÁZAR GONZÁLEZ, Alberto, 28034 Madrid (ES); ESCOBAR PESO, Alejandro, 28034 Madrid (ES); MARCO CONTELLES, José Luis, 28006 Madrid (ES); ALONSO GÓMEZ, José Miguel, 28006 Madrid (ES); LÓPEZ MUÑOZ, Francisco, 28692 Madrid (ES)
(74) Representative: Hoffmann Eitle

(57) **Abstract**

The present invention provides compounds of formula (I) or pharmaceutically acceptable salts or hydrates thereof, as well as pharmaceutical compositions comprising thereof.

The compounds of the invention are useful as neuroprotective agents and, hence, suitable in the treatment or prevention of strokes or cerebral ischaemia.

Advantageously, the compounds of formula (I) are highly potent, thus requiring a remarkable low amount to provide a significant neuroprotective effect.

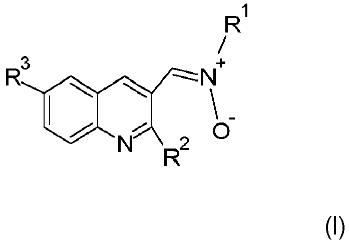

## Description

### Technical Field

The present invention relates to the medical field, particularly to cerebral stroke and ischemia. The present invention provides new quinolylnitrones which show to be highly potent in inducing a neuroprotective effect.

### Background of the Invention

Stroke is a pathology caused by the interruption of the blood supply to the brain, frequently of ischemic nature (87% of cases), mainly caused by a thrombus, but also due to the rupture of a blood vessel (hemorrhagic stroke). By cutting off the supply of oxygen and nutrients, stroke causes damage to the brain tissue. A very severe stroke can cause sudden death, but stroke is more incapacitating than lethal, and constitutes the major cause of neurological disability and the second cause of dementia after Alzheimer's disease. Restoration of the normal circulatory conditions may reverse the damage, but if not, compromised tissue may also turn into infarcted tissue. Thus, to get a proper reperfusion should be the first goal for any ischemic stroke therapy, but other new therapies are sought to extend the benefit of reperfusion therapies, ameliorate the restoration of cellular homeostasis and improve neuroprotection against the damage caused by reperfusion itself, e.g., oxidative stress. This is the case of neuroprotection based on antioxidants such as nitrones, well-known organic molecules showing strong radical scavenging properties.

In this context, in the last decade, great efforts have been made in developing small molecules for the therapy of cerebral ischemia (CI). In this way, new α-phenyl-N-tert-butyl nitrone analogues, (Z)-N-tert-butyl-1-(diethoxyphosphoryl)methanimine oxides, cholesteronitrones, and quinolylnitrones (QNs) have been reported. Particularly, in the development of QNs, it has been demonstrated that (Z)-N-tert-butyl-1-(2-chloro-6-methoxyquinolin-3-yl)methanimine oxide (QN23): 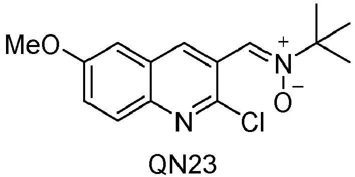 is a potent antioxidant agent, showing strong power to scavenge diverse radical oxygenated species (ROS) and efficient in vitro and in vivo neuroprotective profile.

In spite of the efforts made, however, there is still the need of small molecules with an improved profile.

### Summary of the Invention

The present inventors have found that highly potent neuroprotective agents can be obtained by incorporating at least one aryl moiety at any of the 2- or 6- position of the small molecule QN23.

As it is shown below, the inventors designed several derivatives starting from QN23. On one hand, they substituted the O-methyl group at C6 by the O-propargyl and O-benzyl groups, including in both cases the benzyl (Bn) and the *tert*-butyl (*tert*-Bu) as the *N*-substituent at the nitrone functional motif, a process that resulted in QNs 1-4. Next, it was explored the replacement of the chlorine atom at C2 by: (a) a hydroxyl group, hence QN5; (b) a fluorine atom, resulting in QNs 6,7; (c) arylsulfones such as in QNs 8-11; and (d) a carbon atom implying an aryl group such as those in QNs 12-15. See Scheme 1 below.

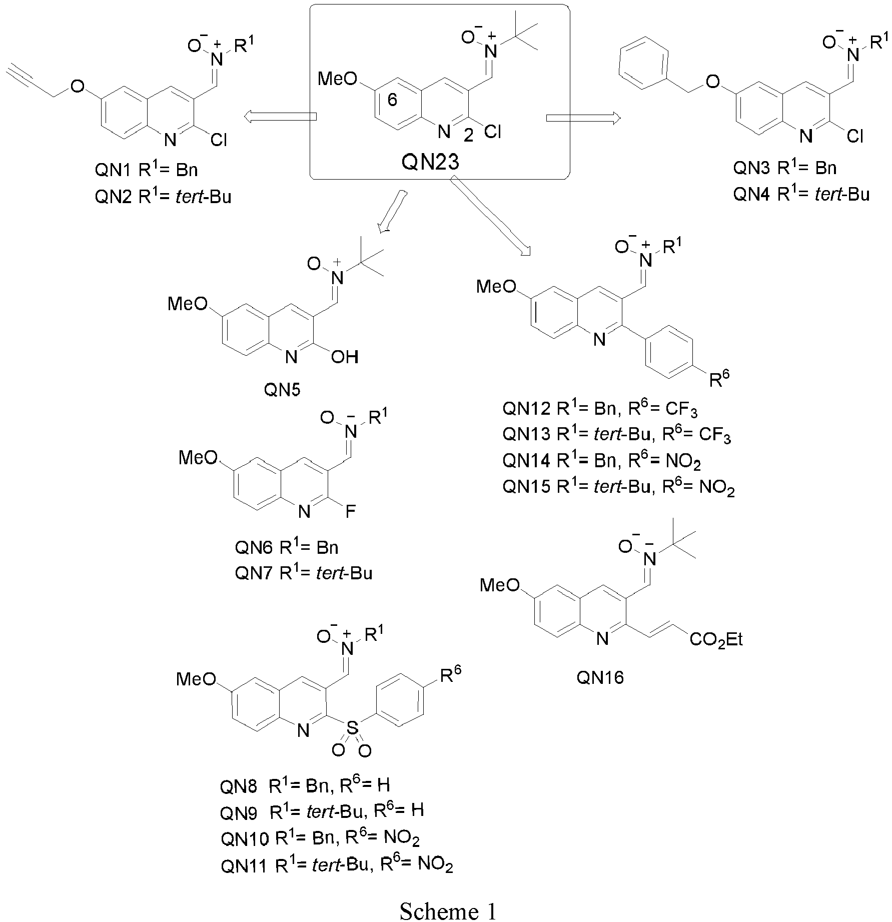

The resulting derivatives were assessed to determine their potential effect on cell viability preservation after an ischemic insult. The results are provided in Table 1 and show that those derivatives incorporating an aryl moiety were more efficient at a substantially lower dose when compared with reference treatments, such as QN23, but also with respect to citicoline or NXY-059. For example, it was found that QN3 provided a higher effect using a 10-fold lower dose when compared to citicoline (reference drug); QN4 provided the best performance at a dose 100-fold lower than the one required to make QN23 provide substantially the same best performance; or QN15, which even improved the neuroprotective efficiency provided by QN23 but, again, using at a remarkably lower dose (about 4-fold lower).

The above means a great advance in the management of therapeutic dosages of this disease: a less amount of the active ingredient is required in the formulation of the pharmaceutical composition to provide the therapeutic effect, avoiding or minimizing potential side-effects of toxicity or insolubility, among others.

Thus, in one aspect the present invention provides a compound of formula (I) or a pharmaceutically salt or hydrate thereof: 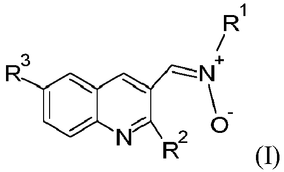 wherein:
R¹ represents (C₁-C₅)alkyl or an aromatic ring system;
R² represents halogen, a known aromatic ring system or -S(O)₂R⁴,
R³ represents -O-(CH₂)ₙ-R⁵;
R⁴ represents a known aromatic ring system;
R⁵ represents (C₁-C₅)alkyl, (C₁-C₅)alkenyl, (C₁-C₅)alkynyl, (C₁-C₅)haloalkyl, or a known aromatic ring system;
n is an integer value selected from 0 and 1;
the term "known aromatic ring system" means an aromatic ring having 5 or 6 members selected from: -CH-, -CR⁶-, -O-, -N-, and -S-;
R⁶ represents halogen, (C₁-C₁₀)alkyl, (C₂-C₁₀)alkenyl, (C₂-C₁₀)alkynyl, (C₁-C₁₀)haloalkyl, (C₂-C₁₀)haloalkenyl, (C₂-C₁₀)haloalkynyl, -O-(C₁-C₁₀)alkyl, - NO₂, -OH, -CN, -COOR⁷, - R⁸C(O)OR⁹, or -C(O)R¹⁰; and
R⁷ to R¹⁰ represents (C₁-C₁₀)alkyl, (C₂-C₁₀)alkenyl, (C₂-C₁₀)alkynyl, and (C₁-C₁₀)haloalkyl;
provided that:
   when R² represents halogen, then R³ represents -O-(CH₂)ₙ-R⁵, being R⁵ an aromatic ring system; and
   when R³ represents -O-(CH₂)ₙ-R⁵, being R⁵ selected from the group consisting of (C₁-Cs)alkenyl, (C₁-C₅)alkynyl, and (C₁-C₅)haloalkyl, then R² represents an aromatic ring system or -S(O)₂R⁴.

Altogether, the compounds of the invention are non-toxic, safe and can be used at a remarkable low dose when compared to the reference compound NXY-059, which is the one currently of reference.

And, therefore, they are suitable for the manufacture of a pharmaceutical composition.

In a second aspect, the present invention provides a pharmaceutical composition comprising a therapeutically effective amount of the compound of formula (I) as defined in the first aspect of the invention, together with one or more pharmacologically acceptable excipients or carriers.

In a third aspect, the present invention provides a compound of formula (I) as defined in the first aspect of the invention for use in therapy.

In a fourth aspect, the present invention provides a compound of formula (I) as defined in the first aspect of the invention for use as neuroprotective agent.

In a final aspect, the present invention provides a compound of formula (I) as defined in the first aspect of the invention for use in the treatment or prevention of cerebral ischaemia or stroke. This aspect can be alternatively be formulated as the use of a compound of formula (I) as defined in the first aspect of the invention in the manufacture of a medicament for the treatment or prevention of cerebral ischaemia or stroke. This aspect can also be formulated as a method for the treatment or prevention of cerebral ischaemia or stroke, the method comprising the step of administering a therapeutically effective amount of a compound of formula (I) as defined in the first aspect of the invention, to a subject in need thereof.

### Brief description of the drawings

FIG. 1 represents the cell viability (C.V.) expressed in AU when the different comparative compounds were tested at different concentrations (C), expressed in µM.
FIG. 2 represents the cell viability (C.V.) expressed in AU when the different compounds of the invention were tested at different concentrations (C), expressed in µM.

### Detailed Description of the Invention

The present invention provides novel compounds of formula (I) with improved properties.

Terms not specifically defined herein should be given the meanings that would be given to them by one of skill in the art in light of the disclosure and the context. As used in the specification, however, unless specified to the contrary, the following terms have the meaning indicated and the following conventions are adhered to.

Throughout the present specification and the accompanying claims the words "comprise" and variations such as "comprises", "comprising" are to be interpreted inclusively. That is, these words are intended to convey the possible inclusion of other elements or integers not specifically recited, where the context allows.

For the purposes of the present invention, any ranges given include both the lower and the upper end-points of the range.

In the groups, radicals, or moieties defined below, the number of carbon atoms is often specified preceding the group, for example, (C₁-C₅)alkyl means an alkyl group or radical having 1 to 5 carbon atoms.

In the present invention, the term "alkyl" is to be understood as an acyclic, straight or branched saturated chain alkyl substituent. Illustrative non-limitative examples are methyl, ethyl, propyl, butyl, hexyl, 1-methylethyl, 1-methylpropyl, 2-methylpropyl or 1,1-dimethylethyl. saturated hydrocarbon chain

In the present invention, the term "alkenyl" is to be understood an aliphatic hydrocarbon group comprising at least one carbon-carbon double bond and which may be straight or branched and comprising about 2 to about 10 carbon atoms in the chain. Non-limiting examples of suitable alkenyl groups include ethenyl, propenyl, n-butenyl, and 3-methylbut-2-enyl.

In the present invention, the term "alkynyl" means an aliphatic hydrocarbon group comprising at least one carbon-carbon triple bond and which may be straight or branched and comprising about 2 to about 10 carbon atoms in the chain. Non-limiting examples of suitable alkynyl groups include ethynyl, propynyl and 2-butynyl.

In the present invention, the term "alkoxy" means an alkyl-O- group in which the alkyl group is as previously described. Non-limiting examples of suitable alkoxy groups include methoxy, ethoxy, n-propoxy and isopropoxy. The alkyl group is linked to an adjacent moiety through the ether oxygen.

In the present invention, the term "halo" means fluoro, chloro, bromo or iodo groups. Preferred are fluoro, chloro or bromo, and more preferred are fluoro and chloro.

In the present invention, the term "halogen" means fluorine, chlorine, bromine or iodine. Preferred are fluorine, chlorine or bromine, and more preferred are fluorine and chlorine.

In the present invention, the term "haloalkyl" means an alkyl as defined above wherein one or more hydrogen atoms on the alkyl is replaced by a halo group defined above. Illustrative examples are monofluoromethyl, difluoromethyl, trifluoromethyl, monochloromethyl, dichloromethyl, trichloromethyl, monobromomethyl, dibromomethyl, tribromomethyl, 1-fluoroethyl, 2-fluoro Ethyl, 2,2-difluoroethyl, 2,2,2-trifluoroethyl, 1-chloroethyl, 2-chloroethyl, 2,2-dichloroethyl, 2,2,2-trichloroethyl, 1-bromoethyl, 2-bromoethyl 2,2-dibromoethyl, 2,2,2-tribromoethyl, 2-iodoethyl, pentafluoroethyl, 3-fluoro-n-propyl, 3-chloro-n-propyl, 3-bromo-n-propyl, 1,3-difluoro-2-propyl, 1,3-dichloro-2-propyl 1,1,1-trifluoro-2-propyl, 1-chloro-3-fluoro-2-propyl, 1,1,1,3,3,3-hexafluoro-2-propyl, 1,1,1, 3,3,3-hexafluoro-2-chloro-2-propyl, 2,2,3,3,3-pentafluoro-n-propyl, heptafluoro-i-propyl, heptafluoro-n-propyl, 4- Fluoro-n-butyl, nonafluoro-n-butyl, nonafluoro-2-butyl, nonafluoro-i-butyl, undecafluoro-n-pentyl, undecafluoro-i-pentyl, undecafluoro-neopentyl, among others.

In the present invention, the term "haloalkenyl" means an alkenyl as defined above wherein one or more hydrogen atoms on the alkyl is replaced by a halo group defined above.

In the present invention, the term "haloalkynyl" means an alkynyl as defined above wherein one or more hydrogen atoms on the alkyl is replaced by a halo group defined above.

The term "substituted" as used herein, means that any one or more hydrogens on the designated atom is replaced with a selection from the indicated group, provided that the designated atom's normal valence is not exceeded, and that the substitution results in a stable compound.

The compounds of the present invention can be in the form of a pure optical isomer, or a mixture of optical isomers in all proportions, or in a form enriched in an optical isomer, and their pharmaceutically acceptable salts, solvates or hydrates.

Solvates of the compounds of the present invention are also contemplated herein. "Solvate" means a physical association of a compound of this invention with one or more solvent molecules. This physical association involves varying degrees of ionic and covalent bonding, including hydrogen bonding. In certain instances the solvate will be capable of isolation, for example when one or more solvent molecules are incorporated in the crystal lattice of the crystalline solid. "Solvate" encompasses both solution-phase and isolatable solvates. Non-limiting examples of suitable solvates include ethanolates, methanolates, and the like. "Hydrate" is a solvate wherein the solvent molecule is H₂O.

The compounds represented by structural formula I may form salts which are also within the scope of this invention. Reference to a compound represented by structural formula I herein is understood to include reference to salts thereof, particularly pharmaceutically acceptable salts thereof, unless otherwise indicated. The term "salt(s)", as employed herein, denotes acidic salts formed with inorganic and/or organic acids, as well as basic salts formed with inorganic and/or organic bases. In addition, when a compound represented by structural formula I contains both a basic moiety, such as, but not limited to, a pyridine or imidazole, and an acidic moiety, such as, but not limited to a carboxylic acid, zwitterions ("inner salts") may be formed and are included within the term "salt(s)" as used herein. Pharmaceutically acceptable salts are preferred, although other salts are also useful. Salts of the compounds represented by structural formula I may be formed, for example, by reacting such as compound with an amount of acid or base, such as an equivalent amount, in a medium such as one in which the salt precipitates or in an aqueous medium followed by lyophilization.

Exemplary acid addition salts include acetates, adipates, alginates, ascorbates, aspartates, benzoates, benzenesulforiates, bisulfates, borates, butyrates, citrates, camphorates, camphorsulfonates, cyclopentanepropionates, digluconates, dodecylsulfates, ethanesulfonates, fumarates, glucoheptanoates, glycerophosphates, hemisulfates, heptanoates, hexanoates, hydrochlorides, hydrobromides, hydroiodides, 2-hydroxyethanesulfonates, lactates, maleates, methanesulfonates, 2-naphthalenesulfonates, nicotinates, nitrates, oxalates, pectinates, persulfates, 3-phenylpropionates, phosphates, picrates, pivalates, propionates, salicylates, succinates, sulfates, sulfonates, tartarates, thiocyanates, toluenesulfonates (also known as tosylates,) undecanoates, and the like.

Exemplary basic salts include ammonium salts, alkali metal salts such as sodium, lithium, and potassium salts, alkaline earth metal salts such as calcium and magnesium salts, salts with organic bases (for example, organic amines) such as benzathines, dicyclohexylamines, hydrabamines (formed with N,N-bis(dehydroabietyl)ethylenediamine), N-methyl-D-glucamines, N-methyl-D-glucamides, t-butyl amines, and salts with amino acids such as arginine, lysine and the like. Basic nitrogen-containing groups may be quartemized with agents such as lower alkyl halides (e.g. methyl, ethyl, propyl, and butyl chlorides, bromides and iodides), dialkyl sulfates (e.g. dimethyl, diethyl, dibutyl, and diamyl sulfates), long chain halides (e.g. decyl, lauryl, myristyl and stearyl chlorides, bromides and iodides), aralkyl halides (e.g. benzyl and phenethyl bromides), and others. All such acid salts and base salts are intended to be pharmaceutically acceptable salts within the scope of the invention and all acid and base salts are considered equivalent to the free forms of the corresponding compounds for purposes of the invention.

Compounds represented by structural formula I, and salts and solvates thereof, may exist in their tautomeric form (for example, as an amide or imino ether). All such tautomeric forms are contemplated herein as part of the present invention.

In one embodiment of the first aspect of the invention, optionally in combination with any of the embodiments provided above or below, the compound of formula (I) is one wherein R² represents halogen or a known aromatic ring system.

In one embodiment of the first aspect of the invention, optionally in combination with any of the embodiments provided above or below, the compound of formula (I) is one wherein R⁵ is selected from (C₁-C₅)alkyl and an aromatic ring system.

In one embodiment of the first aspect of the invention, optionally in combination with any of the embodiments provided above or below, the compound of formula (I) is one wherein the known aromatic ring system has 6 members.

In one embodiment of the first aspect of the invention, optionally in combination with any of the embodiments provided above or below, the compound of formula (I) is one wherein the aromatic ring system has 6 members selected from -CH- and -CR⁶-, R₆ being as defined in claim 1.

In one embodiment of the first aspect of the invention, optionally in combination with any of the embodiments provided above or below, the compound of formula (I) is one wherein R₆ is selected from halogen, (C₁-C₅)haloalkyl, and -NO₂.

In one embodiment of the first aspect of the invention, optionally in combination with any of the embodiments provided above or below, the compound of formula (I) is one wherein R₁ represents (C₁-C₅)alkyl or a benzyl.

In one embodiment of the first aspect of the invention, optionally in combination with any of the embodiments provided above or below, the compound of formula (I) is one wherein R₂ represents halogen or a phenyl group optionally substituted with one or more groups selected from (C₁-C₅)haloalkyl, and -NO₂.

In one embodiment of the first aspect of the invention, optionally in combination with any of the embodiments provided above or below, the compound of formula (I) is one wherein R₃ represents -O-(C₁-C₅)alkyl or a benzyloxy group.

In one embodiment of the first aspect of the invention, optionally in combination with any of the embodiments provided above or below, the compound of formula (I) is one selected from the group consisting of:
(Z)-N-Benzyl-1-(6-(benzyloxy)-2-chloroquinolin-3-yl)methanimine oxide (QN3);
(Z)-N-tert-Butyl-1-(6-(benzyloxy)-2-chloroquinolin-3-yl)methanimine oxide (QN4);
(Z)-N-Benzyl-1-(6-methoxy-2-(phenylsulfonyl)quinolin-3-yl)methanimine oxide (QN8);
(Z)-N-tert-Butyl-1-(6-methoxy-2-(phenylsulfonyl)quinolin-3-yl)methanimine oxide (QN9);
(Z)-N-Benzyl-1-(2-((4-nitrophenyl)sulfonyl)-6-methoxyquinolin-3-yl)methanimine oxide (QN10);
(Z)-N-tert-Butyl-1-(2-((4-nitrophenyl)sulfonyl)-6-methoxyquinolin-3-yl)methanimine oxide (QN11);
(Z)-N-Benzyl-1-(6-methoxy-2-(4-(trifluoromethyl)phenyl)quinolin-3-yl)methanimine oxide (QN12);
(Z)-N-tert-Butyl-1-(6-methoxy-2-(4-(trifluoromethyl)phenyl)quinolin-3-yl)methanimine oxide (QN13);
(*Z*)-*N*-Benzyl-1-(6-methoxy-2-(4-nitrophenyl)quinolin-3-yl)methanimine oxide (QN14); and
(*Z*)-*N*-*tert*-Butyl-1-(6-methoxy-2-(4-nitrophenyl)quinolin-3-yl)methanimine oxide (QN15).

Particularly, the compound of formula (I) is selected from (Z)-N-tert-butyl-1-(6-(benzyloxy)-2-chloroquinolin-3-yl)methanimine oxide (QN4), (*Z*)-*N*-Benzyl-1-(6-methoxy-2-(4-nitrophenyl)quinolin-3-yl)methanimine oxide (QN14), and *(*Z)-*N*-*tert*-Butyl-1-(6-methoxy-2-(4-nitrophenyl)quinolin-3-yl)methanimine oxide (QN15)..

The compound of formula (I) of the invention can be obtained following routine protocols. By the way of illustration, the Examples provided below discloses a way for their obtaining.

Briefly, compounds of the invention QN3 and QN4 were obtained by O-benzylation of 2-chloro-6-hydroxyquinoline-3-carbaldehyde (2) under mild basic reaction conditions (see Scheme 3) and the synthesis of further functionalized compounds of the invention are summarized in Schemes 4 and 5. Briefly, arylsulfonyl-substituted intermediates 7 and 8 were prepared by nucleophilic substitution of 2-chloro-6-methoxyquinoline-3-carbaldehyde (1) using KF and arylsulfonyl chlorides, respectively, and following modifications of the reported procedures. Thus, reaction of carbaldehydes 6-8 with the appropriate *N*-alkyl hydroxylamine hydrochloride led to the expected and QNs 8-11 (Scheme 6), in moderate to excellent yields. 2-Aryl-substituted QNs 12-15 were envisioned through a short reaction sequence including C-C coupling process under Suzuki conditions. Thus, as 2-chloroquinoline 1 was reacted under (Pd₂(dba)₃/PPh₃/*tert*-BuOK) on the more reactive 2-iodo-6-methoxyquinoline-3-carbaldehyde (9), affording the expected aryl-substituted quinolines 10 and 11 with good to excellent yields (Scheme 7).

In a second aspect the present invention provides a pharmaceutical composition comprising the compound of formula (I) as defined in the first aspect of the invention or any of the embodiments provided above.

As used herein, the term "therapeutically effective amount" means that amount of a drug that will elicit the biological or medical response of a tissue, system, animal or human that is being sought, for instance, by a researcher or clinician. Furthermore, the term "therapeutically effective amount" means any amount which, as compared to a corresponding subject who has not received such amount, results in improved treatment, healing, prevention, or amelioration of a disease, disorder, or side effect, or a decrease in the rate of advancement of a disease or disorder. The term also includes within its scope amounts effective to enhance normal physiological function.

The pharmaceutical composition according to the invention can be prepared as a liquid, semi-solid or solid pharmaceutical form, for example in the form of solutions for injections, drops, juices, syrups, sprays, suspensions, tablets, patches, capsules, dressings, suppositories, ointments, creams, lotions, gels, emulsions, aerosols or in multiparticulate form, for example in the form of pills or granules, in appropriate case compressed into tablets, decanted into capsules or suspended in a liquid, and be administered as such.

Pharmaceutically acceptable adjuvants and vehicles that can be used in said compositions are the adjuvants and carriers known to those skilled in the art and commonly used in the preparation of therapeutic compositions, which can be selected, for example, from the group consisting of excipients, fillers, solvents, thinners, surface-active substances, dyes, preservatives, disintegrants, slip agents, lubricants, flavorings and binders.

The selection of physiologically compatible adjuvants and the amount to be used of them depend on the form of administration of the pharmaceutical composition, i.e. oral, subcutaneous, parenteral, intravenous, intraperitoneal, intradermal, intramuscular, intranasal, buccal or rectal. preparations in the form of tablets, dragees, capsules, granules, pills, drops, juices and syrups are suitable preferably for oral administration; solutions, suspensions, easily reconstituted dry preparations and also sprays are suitable preferably for parenteral, topical and inhalation administration.

The compounds according to the invention used in the pharmaceutical composition of according to the invention in a reservoir, in a dissolved form or in a dressing, and in appropriate case having added other agents that favour skin penetration, are preparations suitable for percutaneous administration.

The ways of preparation orally or percutaneously administrable may also release the respective compound from according to the invention in a delayed manner.

On the other hand, it is noted that any of the compounds mentioned as examples throughout the present invention can be used separately or in combination, particularly as adjuvant therapy administered simultaneously, alternatively or successively with respect to a first-line therapy suitable for the treatment of a neurological disease, such as cerebral ischaemia, Alzheimer's disease, Parkinson's disease and amyotrophic lateral sclerosis. In this sense, the quinolylnitrones of formula I administered simultaneously, alternatively or successively with respect to a thrombolytic agent/s and/or thrombectomy procedures, result in particularly suitable therapy for the treatment of cerebral ischaemia, particularly acute cerebral ischaemia.

Thus in one embodiment of the second aspect of the invention, the composition further comprises a thrombolytic agent. The pharmaceutical compositions according to the invention are prepared with the help of means, devices, methods and conventional processes known in the art.

In another embodiment, the invention refers to the pharmaceutical composition as defined above, characterized in that it is in a solid form or in aqueous suspension, in a pharmaceutically acceptable diluent, for administration orally, topically, rectal or parenteral.

In another embodiment, the invention refers to the pharmaceutical composition defined above, where parenteral administration is subcutaneous, intraperitoneal, intradermal, intramuscular or intravenous.

The compounds of the invention can be used as neuroprotective agents. In the context of the invention a compound provides a neuroprotective when, following the protocol provided below in the section of Examples, it is induced a cell viability higher than the one induced by the reoxygenation alone (vehicle-treated group, R24h), which was established as a 0% of neuroprotection.

The compounds of the invention can be used in an adjuvant therapy, being administered simultaneously, alternatively or successively with a first-line therapy suitable for the treatment of cerebral ischaemia or stroke. Particularly, the compounds of the invention can be used in an adjuvant therapy, being administered simultaneously, alternatively or successively with respect to a thrombolytic agent (such as tissue plasminogen activator (tPA) or recombinant tissue plasminogen activator (rtPA)) and/or thrombectomy procedure.

### EXAMPLES

### 1. Design and Synthesis of Quinolylnitrones

### 1.1. Materials

Melting points were determined on a Köffler apparatus. ¹H NMR and ¹³C NMR spectra were recorded in CDCl₃ at 300 MHz and at 75 MHz, respectively, using solvent peaks [CDCl₃: 7.26 (D), 77.2 (C) ppm; D₂O: 4.60 ppm and DMSO-d₆: 2.49 (D), 39.52 (C) ppm] as internal reference. Mass spectra were recorded on a GC/MS spectrometer with an API-ES ionization source. Elemental analyses were performed at CNQO (CSIC, Spain). TLCs were performed on silica F254 and detection by UV light at 254 nm or by charring with either ninhydrin, anisaldehyde or phosphomolybdic-H₂SO₄ reagents. Anhydrous solvents were used in all experiments. Column chromatography was performed on silica gel 60 (230 mesh).

### 1.2. General procedures.

(A) General procedure for the synthesis of nitrones. A solution of the corresponding carbaldehyde (1 mmol), Na₂SO₄ (3 mmol), TEA (2 mmol) and the appropriate hydroxylamine hydrochloride (1.5 mmol) in THF/EtOH (5 mL, 4:1) was heated at 90 °C during 1-6 h under microwave irradiation (MWI). After that time, the solvent was evaporated and the crude mixture was purified on column chromatography using the indicated mixtures of solvents.
(B) General procedure for the synthesis of 6-methoxy-2-(arylsulfonyl)quinoline-3-carbaldehydes 7, 8. A solution of 2-chloro-6-methoxyquinoline-3-carbaldehyde (1) (1 mmol), the corresponding arylsulfonyl chloride (1.2 mmol), and Na₂SO₃ (1.3 mmol) in H₂O (8 mL) was heated at 80 °C in an oil bath for 5 h. After that time, the mixture was diluted with DCM (15 mL), and extracted with NaHCO₃ (5% water solution), brine, and the organic phase was dried over MgSO₄. After filtration and evaporation of the solvent, the crude mixture was purified on column chromatography using the indicated mixtures of solvents. Arylsulfonyl quinolines were obtained as mixtures of the expected products and starting material that we were unable to separate, and were directly used in the next step.
(C) General procedure for the synthesis of 6-methoxy-2-arylquinoline-3-carbaldehydes 10, 11. A solution of 2-iodo-6-methoxyquinoline-3-carbaldehyde (9) (1 mmol), the corresponding arylboronic acid (1.2 mmol), Pd₂dba₃ (5 mol%), PPh₃ (5 mol%), and *tert-BuOK* (1 mmol) in a mixture of toluene/H₂O (5 mL, 4:1) was refluxed during 16 h. Then, the solvent was evaporated and the residue was filtered through a path of Celite. Expected aldehydes were obtained as non-separable mixtures of compounds along with starting material, and therefore directly submitted to the next step without further purification.

### 1.3. Synthesis of compounds of formula (I)

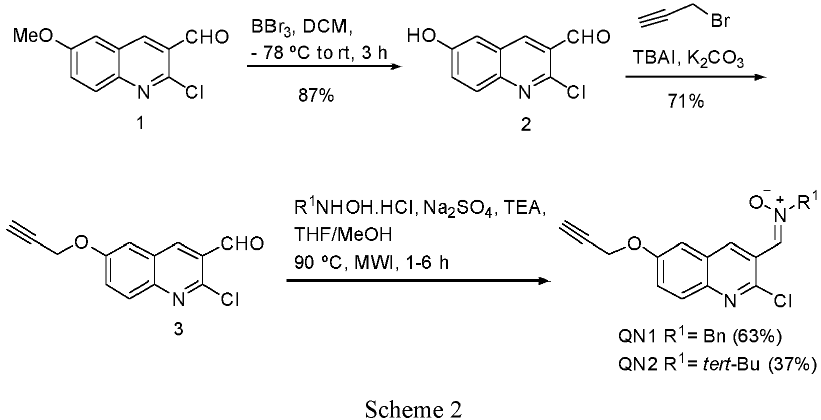

(*Z*)-*N*-Benzyl-1-(2-chloro-6-(prop-2-yn-1-yloxy)quinolin-3-yl)methanimine oxide (QN1). (comparative purpose) Following the General procedure A, the reaction of carbaldehyde 3 (100 mg, 0.408 mmol) with Na₂SO₄ (154 mg, 1.224 mmol), TEA (0.11 mL, 0.816 mmol) and *N-*benzylhydroxylamine hydrochloride (97 mg, 0.612 mmol) in THF/EtOH (5 mL, 4:1) for 1 h, gave after work-up and column chromatography (hexane/AcOEt, 4:1) nitrone QN1 as a white solid (45 mg, 63%): mp 122-3 °C; ¹H NMR (300 MHz, CDCl₃) δ 10.14 (s, 1H), 8.03 (s, 1H), 7.79 (dd, *J=* 9.2, 2.7 Hz, 1H), 7.51-7.27 (m, 6H, H7, C₆H₅), 7.18 (d, *J=* 2.7 Hz, 1H), 5.09 (s, 2H, NCH₂Ph), 4.71 (d, *J=* 2.4 Hz, 2H, OC*H*₂C≡CR), 2.48 (t, *J=* 2.4 Hz, 1H, OCH₂C≡C*H*); ¹³C NMR (75 MHz, CDCl₃) δ 156.6 (C), 146.5 (C), 143.6 (C), 136.6 (CH), 133.1 (C), 130.0 (CH), 129.8 (CH), 129.79 (CH), 129.74 (2 CH C2'), 129.56 (CH), 129.52 (CH), 128.2 (C), 124.7 (CH), 122.9 (C), 108.4 (CH), 78.1 (C), 76.7 (CH), 72.7 (CH₂), 56.5 (CH₂); MS (EI) 350.0 (1) [M⁺]; 315.1 (100) [M⁺-Cl]. HRMS (ESI ACN). Calcd. for C₂₀H₁₅ClN₂O₂: 350.08221. Found: 350.08263. Anal. Calcd. for C₂₀H₁₅ClN₂O₂.1/3H₂O: C, 67.32; H, 4.43; N, 7.85. Found: C, 67.34; H, 4.26; N, 7.91.

(*Z*)-*N*-*tert*-Butyl-1-(2-chloro-6-(prop-2-yn-1-yloxy)quinolin-3-yl)methanimine oxide (QN2). (comparative purpose) Following the General procedure A, the reaction of carbaldehyde 3 (100 mg, 0.408 mmol) with Na₂SO₄ (154 mg, 1.224 mmol), TEA (0.11 mL, 0.816 mmol) and *N-tert-*butylhydroxylamine hydrochloride (77 mg, 0.612 mmol) in THF/EtOH (5 mL, 4:1) for 6 h, after work-up and column chromatography (hexane/AcOEt/DCM, 3:1:1) afforded compound **QN2** as a white solid (37 mg, 37%): mp 106-7 °C; ¹H NMR (300 MHz, CDCl₃) δ 10.27 (s, 1H), 8.21 (s, 1H), 7.82 (d, *J=* 9.2 Hz, 1H), 7.35 (dd, *J=* 9.2, 2.8 Hz, 1H), 7.24 (d, *J=* 2.8 Hz, 1H), 4.73 (d, J= 2.4 Hz, 2H), 2.49 (t, *J=* 2.4 Hz, 1H), 1.61 (s, 9H); ¹³C NMR (75 MHz, CDCl₃) δ 156.6 (C), 147.1 (C), 143.5 (C), 136.3 (CH), 130.0 (CH), 128.4 (C), 125.8 (CH), 124.5 (CH), 123.4 (C), 108.4 (CH), 78.1 (C), 76.7 (CH), 72.9 (C), 56.5 (CH₂), 28.7 (3 × CH₃); MS (EI): 316.1 (2) [M⁺]; 281.1 (32) [M⁺-Cl]; 225.1 (100) [281.1-*tert*-Bu]. HRMS ESI_ACN. Calcd. for C₁₇H₁₇ClN₂O₂: 316.09786. Found: 316.09909. Anal. Calcd. for C₁₇H₁₇ClN₂O₂.½H₂O: C. 62.67; H, 5.57; N, 8.60, Found: C, 62.44; H, 5.29; N, 8.63.

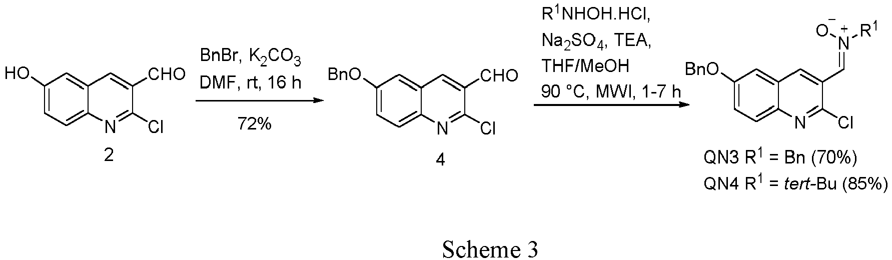

(*Z*)-*N*-Benzyl-1-(6-(benzyloxy)-2-chloroquinolin-3-yl)methanimine oxide (QN3). Following the General procedure A, the reaction of carbaldehyde 4 (70 mg, 0.236 mmol) with N-benzylhydroxylamine hydrochloride (56 mg, 0.353 mmol), Na₂SO₄ (89 mg, 0.708 mmol) and TEA (65 µL, 0.472 mmol) in THF/EtOH (3:0.5 mL), for 1 h, after work-up and column chromatography (hexane/AcOEt, 2:1), gave nitrone QN3, obtained as a white solid (65 mg, 70%): mp 182-3 °C; ¹H NMR (300 MHz, CDCl₃) δ 10.09 (s, 1H), 8.02 (s, 1H), 7.79 (d, *J=* 9.2 Hz, 1H), 7.53-7.23 (m, 11H, H7, 2 C₆H₅), 7.11 (d, *J=* 2.8 Hz, 1H), 5.08 (s, 4H); ¹³C NMR (75 MHz, CDCl₃) δ 157.9 (C), 146.2 (C), 143.5 (C), 136.5 (CH), 133.1 (2 C), 130.0 (2 CH), 129.9 (CH), 129.8 (2 CH), 129.7 (CH), 129.5 (2 CH), 129.1 (2 CH), 128.7 (CH), 128.4 (C), 127.9 (CH), 125.1 (CH), 122.8 (C), 108.0 (CH), 72.7 (CH₂), 70.8 (CH₂); MS (EI): 402.1 (5) [M⁺]; 367 (100) [M⁺-Cl]. HRMS ESI_ACN. Calcd. for C₂₄H₁₉ClN₂O₂: 402.11351. Found: 402.11419. Anal. Calcd. for C₂₄H₁₉ClN₂O₂: C, 70.50; H, 4,85; N, 6.85. Found: C, 70.39; H, 4.79; N, 6.81.

(*Z*)-*N*-*tert*-Butyl-1-(6-(benzyloxy)-2-chloroquinolin-3-yl)methanimine oxide (QN4). Following the General procedure A, the reaction of carbaldehyde 4 (70 mg, 0.236 mmol) with *N-tert-*butylhydroxylamine hydrochloride (44 mg, 0.353 mmol), Na₂SO₄ (89 mg, 0.708 mmol) and TEA (65 µL, 0.472 mmol) in THF/EtOH (3:0.5 mL), for 6 h, gave after work-up and column chromatography (hexane/AcOEt, 7:3) nitrone QN4, obtained as a white solid (73 mg, 85%): mp 205-6 °C; ¹H NMR (300 MHz, CDCl₃) δ 10.19 (s, 1H), 8.19 (s, 1H), 7.80 (d, *J*= 9.1 Hz, 1H), 7.45-7.24 (m, 6H), 7.13 (d, *J=* 2.8 Hz, 1H), 5.10 (s, 2H), 1.60 (s, 9H); ¹³C NMR (75 MHz, CDCl₃) δ 157.9 (C), 146.8 (C), 143.3 (C), 136.5 (CH), 136.2 (C), 129.9 (CH), 129.1 (2 CH), 128.67 (CH), 128.63 (C), 127.8 (2 CH), 125.8 (CH), 124.8 (CH), 123.3 (C), 108.1 (CH), 72.8 (C), 70.7 (CH₂), 28.7 (3 × CH₃); MS (EI): 367.1 (2) [M⁺]; 333.1 (37) [M⁺-Cl]. HRMS ESI_ACN. Calcd. for C₂₁H₂₁ClN₂O₂: 368.12916. Found: 368.12987. Anal. Calcd. for C₂₁H₂₁ClN₂O₂: C, 68.35; H, 5,74; N, 7.59. Found: C, 68.22; H, 5.74; N, 7.58.

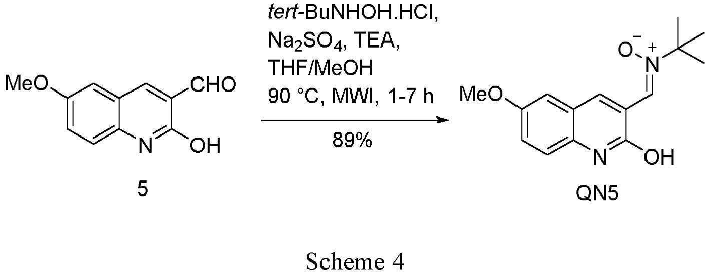

(*Z*)-*N*-*tert*-Butyl-1-(2-hydroxy-6-methoxyquinolin-3-yl)methanimine oxide (QN5). (comparative purpose). Following the General procedure A, the reaction of commercial compound 5 (50 mg, 0.153 mmol) with *N*-*tert*-butylhydroxylamine hydrochloride (30 mg, 0.229 mmol), Na₂SO₄ (60 mg, 0.459 mmol) and TEA (42 , 0.306 mmol) in THF (2 mL), for 6 h, after work-up and column chromatography (DCM/AcOEt, 1:2), gave nitrone QN5 as a yellow solid (40 mg, 89%): mp 172-3 °C; ¹H NMR (300 MHz, CDCl₃) δ 11.77 (br s, 1H), 10.06 (s, 1H), 8.26 (s, 1H), 7.28-7.15 (m, 1H), 7.09 (dd, *J=* 8.9, 2.6 Hz, 1H), 6.99 (d, *J=* 2.6 Hz, 1H), 3.76 (s, 3H, OCH₃), 1.59 (s, 9H); ¹³C NMR (75 MHz, CDCl₃) δ 162.7 (C), 155.8 (C), 137.8 (CH), 133.1 (C), 125.3 (CH), 122.7 (C), 121.6 (CH), 121.4 (C), 117.1 (CH), 110.3 (CH), 72.1 (C), 56.0 (CH₃), 28.7 (3 × CH₃); MS (EI): 274.1 (62) [M⁺]. HRMS ESI_ACN. Calcd. for C₁₅H₁₈N₂O₃: 274.13174. Found: 274.13130. Anal. Calcd for C₁₅H₁₈N₂O₃: C, 65.68; H, 6,61; N, 10.21. Found: C, 65.39; H, 6.58; N, 9.97.

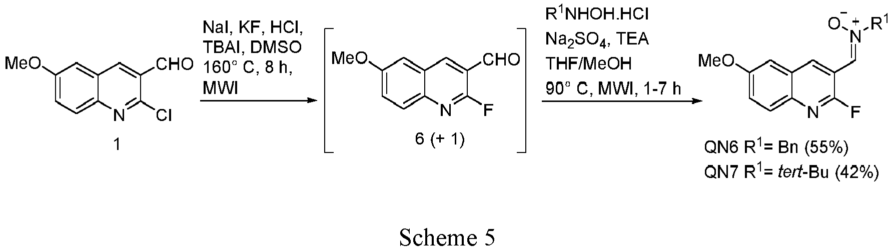

(*Z*)-*N*-Benzyl-1-(6-(methoxy)-2-fluoroquinolin-3-yl)methanimine oxide (QN6) (comparative purpose). A solution of 2-chloro-6-methoxyquinoline-3-carbaldehyde (1) (70 mg, 0.317 mmol), NaI (190 mg, 1.267 mmol), KF (147 mg, 2.536 mmol), and catalytic amount of HCl and TBAI in DMSO (1 mL), was heated at 160 ⁰C for 8 h under MWI. Then, the mixture was diluted with AcOEt and washed with H₂O (3x10 mL). The organic phase was washed with brine and dry over MgSO₄. After evaporation of the solvent, the crude mixture was purified by column chromatography (hexane/AcOEt, 7:3) to yield a mixture of major 2-fluoro-6-methoxyquinoline-3-carbaldehyde (6) {¹H NMR (300 MHz, CDCl₃) δ 10.32 (s, 1H, CHO), 8.35 (s, 1H, H4), 7.88 (dd, *J=* 9.2, 1.0 Hz, 1H, H8), 7.43 (dd, *J=* 9.2, 2.8 Hz, 1H), 7.10 (dd, *J=* 2.8, 1.0 Hz, 1H), 3.93 (s, 3H, OCH₃); MS (EI): 206.1 (100) [M+1]} and minor starting material 1, that we could not separate (55 mg of a mixture of compounds 9/1 in 1:0.3 ¹H NMR ratio. Compound 6, obtained in 65 % based on the ¹H NMR spectrum, was used in the next steps without further purification. Following the General procedure A, the reaction of the mixture 6+1 (60 mg) with *N*-benzylhydroxylamine hydrochloride (70 mg, 0.439 mmol), Na₂SO₄ (111 mg, 0.879 mmol) and TEA (81 µL, 0.586 mmol) in THF/EtOH (3:0.5 mL), for 1 h, after work-up and column chromatography (hexane/AcOEt/Et₂O, 4:1:1) gave nitrone QN6 as a white solid (50 mg, 55%): mp 197-9 °C; ¹H NMR (300 MHz, CDCl₃) δ 10.08 (d, *J=* 9.8 Hz, 1H), 7.77 (s, 1H), 7.69 (d, *J=* 9.1 Hz, 1H), 7.49-7.22 (m, 6H), 7.08 (d, *J=* 2.8 Hz, 1H), 5.07 (s, 2H), 3.82 (s, 3H); ¹³C NMR (75 MHz, CDCl₃) δ 158.3 (C, d, *J=* 2.2 Hz), 156.5 (d, *J=* 242 Hz, C), 140.1 (C, d, *J=* 68.4 Hz), 138.5 (d, *J=* 3.4 Hz, CH), 133.1 (C), 129.7 (2 CH), 129.5 (2 CH), 129.2 (CH), 128.4 (C), 126.6 (CH), 124.4 (CH), 114.1 (C, d, *J=* 28.5 Hz), 107.2 (CH), 72.4 (CH₂), 56.0 (CH₃); MS (EI): 310.1 (100) [M⁺]. HRMS ESI_ACN Calcd. for C₁₈H₁₅FN₂O₂: 310.11176. Found: 310.11076. Anal. Calcd for C₁₈H₁₅FN₂O₂: C, 69.67; H, 4,87; N, 9.03. Found: C, 69.52; H, 5.01; N, 9.04.

(*Z*)-*N*-*tert*-Butyl-1-(6-(methoxy)-2-fluoroquinolin-3-yl)methanimine oxide (QN7). (comparative purpose) Following the General procedure A, the reaction of mixture 6+1 (100 mg) with *N-tert-*butylhydroxylamine hydrochloride (88 mg, 0.731 mmol), Na₂SO₄ (184 mg, 1.461 mmol) and TEA (135 µL, 0.974 mmol) in THF (4 mL), for 6 h, after work-up and column chromatography (hexane/AcOEt/MeOH, 10:5:1) gave nitrone QN7, as a white solid (57 mg, 42%): mp 156-7 °C; ¹H NMR (300 MHz, CDCl₃) δ 10.26 (d, *J=* 9.8 Hz, 1H), 7.96 (s, 1H), 7.77 (d, *J=* 9.2 Hz, 1H), 7.37 (dd, *J=* 9.2, 2.8 Hz, 1H), 7.18 (s, 1H), 3.89 (s, 3H), 1.65 (s, 9H); ¹³C NMR (75 MHz, CDCl₃) δ 158.2 (C, d, *J=* 2.0 Hz), 156.8 (d, *J=* 239 Hz, C), 140.7 (C, d, *J=* 17.4 Hz), 138.2 (d, *J=* 3.5 Hz, CH), 129.2 (CH), 128.5 (C), 124.0 (C), 122.4 (CH), 114.5 (C, d, *J=* 27.7 Hz), 107.2 (CH), 72.6 (C), 55.9 (CH₃), 28.7 (3 x CH₃); MS (EI): 276.1 (57) [M⁺]. HRMS ESI_ACN. Calcd. for C₁₅H₁₇FN₂O₂: 276.12741. Found: 276.12834. Anal. Calcd. C₁₅H₁₇FN₂O₂: C, 63.15; H, 6.36; N, 9.82. Found: C, 63.35; H, 6.15; N, 9.74.

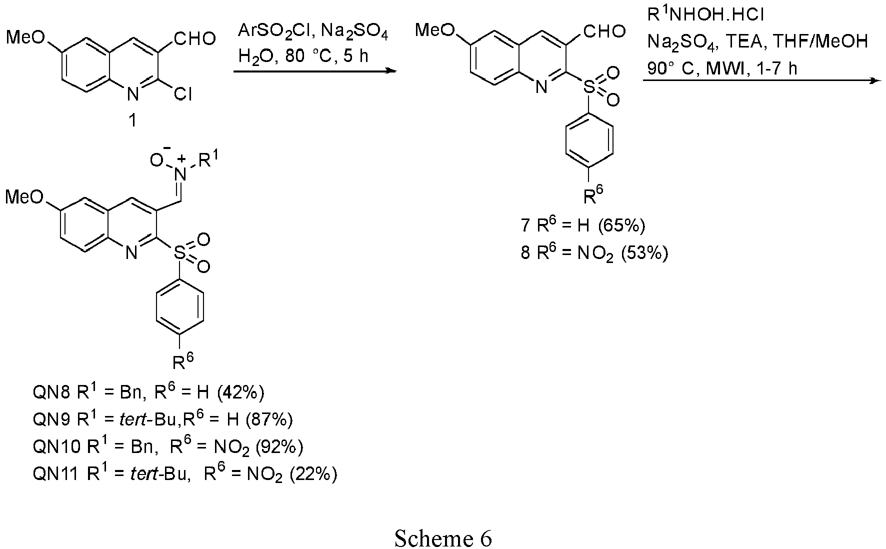

(*Z*)-*N*-Benzyl-1-(6-methoxy-2-(phenylsulfonyl)quinolin-3-yl)methanimine oxide (QN8). Following the General procedure B, the reaction of 2-chloro-6-methoxyquinoline-3-carbaldehyde (1) (42 mg, 0.189 mmol) with phenylsulfonyl chloride (72 µL, 0.567 mmol) and Na₂SO₃ (71 mg, 0.567 mmol) in H₂O (0.7 mL), after purification on column chromatography (hexane/AcOEt, 2:1) gave a mixture of unreacted compound 1 and 6-methoxy-2-(phenylsulfonyl)quinoline-3-carbaldehyde (7) (40 mg, 65%, yield estimated on the ¹H NMR basis spectrum), as a thick gum {¹H NMR (300 MHz, CDCl₃) (for major compound 7) δ 11.09 (s, 1H), 8.69 (s, 1H), 8.12-7.96 (m, 2H), 7.85 (d, *J=* 9.2 Hz, 1H), 7.64-7.48 (m, 3H), 7.42 (dd, *J*= 9.2, 2.7 Hz, 1H), 7.14 (d, *J=* 2.7 Hz, 1H), 3.89 (s, 3H); MS (EI): 327.1 (5) [M⁺]}. Following the General procedure A, the reaction of mixture 1+7 (200 mg, 0.611 mmol) with *N*-benzylhydroxylamine hydrochloride (146 mg, 0.917 mmol), Na₂SO₄ (260 mg, 1.835 mmol) and TEA (170 µL, 1.223 mmol) in THF (4 mL), for 1 h and work-up, after purification on column chromatography (hexane/AcOEt, 2:1) gave the expected nitrone QN8 as a light yellow solid (97 mg, 42%): mp 164-6 °C; ¹H NMR (300 MHz, CDCl₃) δ 10.29 (s, 1H), 8.89 (s, 1H), 7.87 (dd, *J=* 8.4, 1.4 Hz, 1H), 7.69-7.25 (m, 11H), 7.04 (d, *J=* 2.7 Hz, 1H), 5.12 (s, 2H), 3.82 (s, 3H); ¹³C NMR (75 MHz, CDCl₃) δ 160.5 (C), 152.5 (C), 141.6 (C), 139.1 (C), 136.3 (CH), 134.1 (C), 133.3 (CH), 131.6 (CH), 130.3 (C), 130.0 (2 CH), 129.7 (2 CH), 129.5 (2 CH), 129.4 (2 CH), 129.2 (CH), 128.4 (CH) 125.3 (CH), 121.4 (C), 106.1 (CH), 73.0 (CH₂), 56.1 (CH₃); MS (EI): 432.1 (3) [M⁺], 291.1 (100) [M⁺-SO₂Ph]. HRMS ESI_ACN. Calcd. for C₂₄H₂₀N₂O₄S: 432.11438. Found: 432.11454. Anal. Calcd. for C₂₄H₂₀N₂O₄S: C, 66.65; H, 4.66; N, 6.48; S, 7.41. Found: C, 66.88; H, 4.88; N, 6.74; S, 7.26.

(*Z*)-*N*-*tert*-Butyl-1-(6-methoxy-2-(phenylsulfonyl)quinolin-3-yl)methanimine oxide (QN9). Following the General procedure A, the reaction of mixture 1+7 (70 mg, 0.214 mmol) with *N-tert-*butylhydroxylamine hydrochloride (90 mg, 0.321 mmol), Na₂SO₄ (180 mg, 0.642 mmol) and TEA (126 µL, 0.428 mmol) in THF (5 mL), for 6 h and work-up, after column chromatography (hexane/AcOEt, 2:1) gave nitrone QN9 obtained as a light yellow solid (80 mg, 87%): mp 137-8 °C; ¹H NMR (300 MHz, CDCl₃) δ 10.39 (s, 1H), 9.04 (s, 1H), 8.00-7.90 (m, 2H), 7.70-7.41 (m, 4H), 7.27 (dd, *J=* 9.2, 2.7 Hz, 1H), 7.07 (d, *J=* 2.7 Hz, 1H), 3.83 (s, 3H), 1.62 (s, 9H); ¹³C NMR (75 MHz, CDCl₃) δ 160.4 (C), 152.9 (C), 141.4 (C), 139.2 (C), 136.0 (CH), 134.1 (CH), 131.5 (CH), 130.5 (C), 129.6 (2 CH), 129.1 (2 CH), 124.9 (CH), 124.3 (CH), 122.0 (C), 106.1 (CH), 73.1 (C), 28.7 (3 × CH₃); MS (EI): 398.1 (3) [M⁺], 257.1 (27) [M⁺-SO₂Ph]. HRMS ESI_ACN. Calcd. for C₂₁H₂₂N₂O₄S: 398.13003. Found: 398.13047. Anal. Calcd. for C₂₁H₂₂N₂O₄S: C, 62.36; H, 5.65; N, 6.93; S, 7.93 Found: C, 62.49; H, 5.54; N, 6.75; S, 7.84.

(*Z*)-*N*-Benzyl-1-(2-((4-nitrophenyl)sulfonyl)-6-methoxyquinolin-3-yl)methanimine oxide (QN10). Following the General procedure B, the reaction of 2-chloro-6-methoxyquinoline-3-carbaldehyde (1) (200 mg, 0.904 mmol) with 4-nitrobenzenesulfonyl chloride (239 mg, 1.085 mmol) and Na₂SO₃ (148 mg, 1.175 mmol) in H₂O (3 mL), after purification on column chromatography (hexane/AcOEt, 4:1), afforded 6-methoxy-2-((4-nitrophenyl)sulfonyl)quinoline-3-carbaldehyde (8) (178 mg, 53%, yield estimated on the ¹H NMR basis spectrum), a thick yellow gum, as a mixture of non-separable compounds 8 and 1 [¹H NMR (300 MHz, CDCl₃) (for compound 8) δ 10.55 (d, *J=* 0.7 Hz), 8.64 (d, *J=* 0.7 Hz, 1H), 8.42 (d, *J=* 8.7 Hz, 2H), 8.30 (d, *J*= 8.7 Hz, 2H), 7.96 (dd, *J=* 9.3, 0.7 Hz, 1H), 7.52 (dd, *J=* 9.3, 2.8Hz, 1H), 7.19 (d, *J*= 2.8 Hz, 1H)]. Following the General procedure A, the reaction of mixture 1+8 (90 mg, 0.410 mmol) with N-benzylhydroxylamine hydrochloride (98 mg, 0.615 mmol), Na₂SO₄ (116 mg, 0.821 mmol) and TEA (170 µL, 1.231 mmol) in THF (3 mL), for 1 h, after work-up and purification on column chromatography (DCM/AcOEt, 5:1), gave nitrone QN10 as a yellow solid (80 mg, 92%): mp 175-7 °C; ¹H NMR (300 MHz, CDCl₃) δ 10.31 (s, 1H), 8.79 (s, 1H), 8.30 (d, *J=* 9.0 Hz, 2H), 8.10 (d, *J=* 9.0 Hz, 2H), 7.62-7.22 (m, 7H), 7.06 (d, *J=* 2.0 Hz, 1H), 5.14 (s, 2H), 3.83 (s, 3H); ¹³C NMR (75 MHz, CDCl₃) δ 160.7 (C), 151.7 (C), 151.2 (C), 144.7 (C), 141.2 (C), 136.4 (CH), 133.1 (C), 131.4 (2 CH), 131.3 (C), 130.4 (2 CH), 129.9 (CH), 129.8 (CH), 129.5 (2 CH), 127.5 (CH), 125.6 (CH), 124.1 (2 CH), 121.3 (C), 106.1 (CH), 73.1 (CH₂), 56.2 (CH₃); MS (EI): 477.1 (1) [M⁺], 291.1 (100) [M⁺-SO₂Ar]. HRMS ESI_ACN. Calcd. for C₂₄H₁₉N₃O₆S: 477.09946. Found: 477.09948. Anal. Calcd. for C₂₄H₁₉N₃O₆S: C, 59.25; H, 4.14; N, 8.64; S, 6.59. Found: C, 59.19; H, 4.12; N, 8.74; S, 6.54.

(*Z*)-*N*-*tert*-Butyl-1-(2-((4-nitrophenyl)sulfonyl)-6-methoxyquinolin-3-yl)methanimine oxide (QN11). Following the General procedure A, the reaction of mixture 1+8 (116 mg, 0.525 mmol), with *N*-*tert*-butylhydroxylamine hydrochloride (98 mg, 0.615 mmol), Na₂SO₄ (98 mg, 0.791 mmol) and TEA (220 µL, 1.550 mmol) in THF (5 mL), for 6 h, after work-up and purification on column chromatography (DCM/AcOEt, 10:1), gave nitrone QN11 as a yellow solid (51 mg, 22%): mp 144-5 °C; ¹H NMR (300 MHz, CDCl₃) δ 10.41 (s, 1H), 8.99 (s, 1H), 8.36 (d, *J=* 8.9 Hz, 2H), 8.18 (d, *J=* 8.9 Hz, 2H), 7.52 (d, *J=* 9.2 Hz, 1H), 7.26 (dd, *J=* 9.2, 2.8 Hz, 1H), 7.09 (d, J= 2.8 Hz, 1H), 3.84 (s, 3H), 1.64 (s, 9H); ¹³C NMR (75 MHz, CDCl₃) δ 160.6 (C), 152.2 (C), 151.2 (C), 144.8 (C), 141.1 (C), 136.1 (CH), 131.4 (2 CH), 131.2 (CH), 130.6 (C), 125.2 (CH), 124.1 (2 CH), 123.5 (CH), 121.9 (C), 106.2 (CH), 73.3 (C), 56.1 (CH₃), 28.7 (3 x CH₃); MS (EI): 443.1 (3) [M⁺], 257.1 (32) [M⁺-SO₂Ar]. HRMS ESI_ACN. Calcd. for C₂₁H₂₁N₃O₆S: 443.11511. Found: 443.11544. Anal. Calcd. for C₂₁H₂₁N₃O₆S: C, 56.88; H, 4.77; N, 9.48; S, 7.23 Found: C, 56.68; H, 4.88; N, 9.41; S, 7.06.

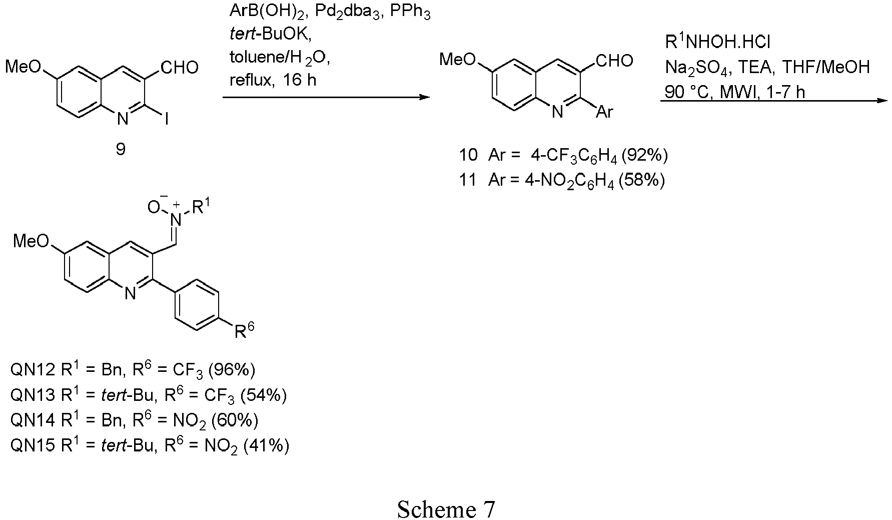

6-Methoxy-2-(4-trifluoromethylphenyl)quinoline-3-carbaldehyde (10). Following the General procedure C, the reaction of compound 9 (50 mg, 0.159 mmol), *p*-trifluoromethylphenylboronic acid (36 mg, 0.191 mmol), *tert-BuOK* (18 mg, 0.159 mmol), Pd₂dba₃ (7 mg, 0.007 mmol) and PPh₃ (2 mg, 0.007 mmol) in toluene/H₂O (4+1 mL), after purification on column chromatography (hexane/AcOEt, 5:1), gave compound 10 (48 mg, 92%) was obtained as a yellow solid: ¹H NMR (300 MHz, CDCl₃) δ 10.09 (s, 1H), 8.68 (s, 1H), 8.03 (d, *J=* 9.2 Hz, 1H), 7.74 (s, 4H), 7.48 (dd, *J* = 9.2, 2.8 Hz, 1H), 7.19 (s, 1H), 3.92 (s, 3H). HRMS ESI_ACN. Calcd. for C₁₈H₁₂NF₃O₂: 331.08201. Found: 331.08222.

(*Z*)-*N*-Benzyl-1-(6-methoxy-2-(4-(trifluoromethyl)phenyl)quinolin-3-yl)methanimine oxide (QN12). Following the General procedure A, the reaction of compound 11 (100 mg, 0.302 mmol) with *N*-benzylhydroxylamine hydrochloride (71 mg, 0.453 mmol), Na₂SO₄ (86 mg, 0.604 mmol) and TEA (120 µL, 0.906 mmol) in THF (3 mL), for 1 h, after purification on column chromatography (DCM/AcOEt, 10:1), gave nitrone QN12 as a yellow solid (120 mg, 96%): mp 234-5 °C; ¹H NMR (300 MHz, CDCl₃) δ 10.15 (s, 1H), 7.88 (d, *J=* 9.2 Hz, 1H), 7.56 (d, *J=* 8.0 Hz, 2H), 7.44 (d, *J=* 8.0 Hz, 2H), 7.37-7.22 (m, 6H), 7.19 (d, *J=* 4.8 Hz, 1H), 7.13 (d, *J=* 2.7 Hz, 1H), 4.95 (s, 2H), 3.87 (s, 3H); ¹³C NMR (75 MHz, CDCl₃) δ 158.8 (C), 155.0 (C), 143.9 (C), 143.9 (C), 143.2 (C), 134.7 (CH), 132.8 (C), 131.3 (CH), 131.0 (CH), 130.2 (2 CH), 130.0 (2 CH), 129.8 (2 CH), 129.5 (2 CH), 128.5 (C), 125.93, 125.88, 125.83, 125.78 (C, q, *J=* 15.0 Hz), 124.6 (CH), 122.4 (C), 106.4 (CH), 72.2 (CH₂), 56.0 (CH₃); MS (EI): 436.1 (29) [M⁺]. HRMS ESI_ACN. Calcd. for C₂₅H₁₉F₃N₂O₂: 436.13986. Found: 436.13835. Anal. Calcd. for C₂₅H₁₉F₃N₂O₂: C, 67.87; H, 4.48; N, 6.33. Found: C, 67.90; H, 4.65; N, 6.52.

(Z)-N-tert-Butyl-1-(6-methoxy-2-(4-(trifluoromethyl)phenyl)quinolin-3-yl)methanimine oxide (QN13). Following the General procedure A, the reaction of compound 11 (120 mg, 0.363 mmol) with *N*-*tert*-butylhydroxylamine hydrochloride (68 mg, 0.544 mmol), Na₂SO₄ (103 mg, 0.726 mmol) and TEA (150 µL, 1.089 mmol) in THF (3 mL), for 6 h, after purification on column chromatography (DCM/AcOEt, 7:1), gave nitrone QN13 as a yellow solid (78 mg, 54%): mp 192-4 °C; ¹H NMR (300 MHz, CDCl₃) δ 10.25 (s, 1H), 7.91 (ddd, *J=* 9.2, 2.8, 1.2 Hz, 1H), 7.72 (d, *J=* 9.1 Hz, 2H), 7.65 (d, *J=* 9.1 Hz, 2H), 7.59 (s,1H), 7.34 (dd, *J* = 9.2, 2.8 Hz, 1H), 7.14 (dd, *J* = 2.8, 1.2 Hz, 1H), 3.87 (s, 3H), 1.45 (s, 9H); ¹³C NMR (75 MHz, CDCl₃) δ 158.8 (C), 155.3 (C), 143.8 (3 C), 134.7 (CH), 131.0 (CH), 130.9 (2 CH), 130.4 (2 CH), 128.6 (C), 127.0 (CH), 126.7 (q, *J* = 270.5 Hz, C), 125.9, 125.9, 125.8, 125.8 (C, q, *J=* 15.0 Hz), 124.3 (CH), 122.9 (C), 106.4 (CH), 72.3 (C), 56.0 (CH₃), 28.6 (3 x CH₃). HRMS ESI_ACN. Calcd. for C₂₂H₂₁F₃N₂O₂: 402.15551. Found: 402.15507. Anal. Calcd. for C₂₂H₂₁F₃N₂O₂: C, 64.94; H, 5.33; N, 6.90. Found: C, 64.77; H, 5.32; N, 7.17.

6-Methoxy-2-(4-nitrophenyl)quinoline-3-carbaldehyde (11). Following the General procedure C, the reaction of commercial 2-iodo-6-methoxyquinoline-3-carbaldehyde 9 (100 mg, 0.452 mmol), *p*-nitrophenylboronic acid (90 mg, 0.542 mmol), *tert*-BuOK (50 mg, 0.452 mmol), Pd₂dba₃ (21 mg, 0.022 mmol) and PPh₃ (18 mg, 0.068 mmol) in toluene/H₂O (4+1 mL), after purification on column chromatography (hexane/AcOEt, 8:2), gave a mixture of 9+11 [¹H NMR (300 MHz, CDCl₃) (major compound 10) [δ 10.17 (s, 1H), 8.77 (s, 1H), 8.42 (dd, *J=* 9.0, 2.3 Hz, 2H), 8.11 (d, *J=* 9.2 Hz, 1H), 7.86 (dd, *J=* 9.0, 2.3 Hz, 2H), 7.57 (dd, *J=* 9.2, 1.9 Hz, 1H), 7.26 (m, 1H), 4.00 (s, 3H)] (81 mg, 58%, yield estimated by ¹H NMR in the reaction mixture) was obtained as a non-separable mixture with starting material. The mixture of compounds was submitted to the next step without further purification.

(*Z*)-*N*-Benzyl-1-(6-methoxy-2-(4-nitrophenyl)quinolin-3-yl)methanimine oxide (QN14). Following the General procedure A, the reaction of mixture 9+10 (100 mg, 0.325 mmol) with N-benzylhydroxylamine hydrochloride (77 mg, 0.487 mmol), Na₂SO₄ (138 mg, 0.975 mmol) and TEA (90 µL, 0.650 mmol) in THF (3.2 mL), for 1 h, after purification on column chromatography (DCM/AcOEt, 2:1), gave nitrone QN14 as a yellow solid (80 mg, 60%): mp 251-3 °C; ¹H NMR (300 MHz, CDCl₃) δ 10.13 (s, 1H), 8.15 (d, *J=* 8.7 Hz, 2H), 7.88 (d, *J=* 9.2 Hz, 1H), 7.51 (d, J= 8.7 Hz, 2H), 7.39-7.24 (m, 6H), 7.18-7.16 (m, 1H), 7.14-7.12 (m, 1H), 4.96 (s, 2H), 3.88 (s, 3H); ¹³C NMR (75 MHz, CDCl₃) δ 159.1 (C), 153.9 (C), 148.0 (C), 146.0 (C), 143.9 (C), 134.8 (CH), 132.8 (C), 131.0 (2 CH), 130.9 (2 CH), 130.0 (CH), 129.8 (CH), 129.7 (2 CH), 129.6 (CH), 128.7 (C), 124.8 (CH), 124.0 (2 CH), 122.3 (C), 106.3 (CH), 72.3 (CH₂), 56.1 (CH₃); MS (EI): 413.1 (23) [M⁺], 291.1 (100) [M⁺-C₆H₄NO₂]. HRMS ESI_ACN. Calcd. for C₂₄H₁₉N₃O₄: 413.13756.

Found: 413.12755. Anal. Calcd. for C₂₄H₁₉N₃O₄: C, 64.55; H, 4.42; N, 9.22. Found: C, 64.50; H, 4.33; N, 8.99.

(*Z*)-*N*-*tert*-Butyl-1-(6-methoxy-2-(4-nitrophenyl)quinolin-3-yl)methanimine oxide (QN15). Following the General procedure A, the reaction of mixture 9+10 (100 mg, 0.325 mmol) with N-*tert*-butylhydroxylamine hydrochloride (61 mg, 0.487 mmol), Na₂SO₄ (138 mg, 0.975 mmol) and TEA (90 µL, 0.650 mmol) in THF (3.2 mL), for 6 h; after purification on column chromatography (DCM/AcOEt, 4:1), gave nitrone QN15, as a yellow solid (45 mg, 41%): mp 189-191°C; ¹H NMR (300 MHz, CDCl₃) δ 10.23 (s, 1H), 8.32 (d, *J=* 8.8 Hz, 2H), 7.91 (d, *J=* 9.2 Hz, 1H), 7.74 (d, *J*= 8.8 Hz, 2H), 7.56 (s, 1H), 7.36 (dd, *J=* 9.2, 2.8 Hz, 1H), 7.14 (d, *J=* 2.8 Hz, 1H), 3.87 (s, 3H), 1.46 (s, 9H); ¹³C NMR (75 MHz, CDCl₃) δ 159.0 (C), 154.2 (C), 148.3 (C), 146.6 (C), 143.8 (C), 134.8 (CH), 131.1 (2 CH), 131.0 (CH), 128.8 (C), 126.6 (CH), 124.6 (CH), 124.1 (2 CH), 122.9 (C), 106.3 (CH), 72.4 (C), 56.0 (CH₃), 28.6 (3 x CH₃); MS (EI): 379.1 (38) [M⁺], 322.1 (80) [M⁺-*tert-*Bu]. HRMS ESI_ACN. Calcd. for C₂₁H₂₁N₃O₄: 379.15321. Found: 379.11535. Anal. Calcd. for C₂₁H₂₁N₃O₄: C, 66.48; H, 5.58; N, 11.08. Found C, 66.34; H, 5.46; N, 10.37.

(*Z*)-*N*-*tert*-Butyl-1 -(2-((E)-3 -ethoxy-3 -oxoprop-1 -en-1 -yl)-6-methoxyquinolin-3 -yl)methanimine oxide (QN16). Following the General procedure A, the reaction of compound 12 (80 mg, 0.280 mmol) with *N*-*tert*-butylhydroxylamine hydrochloride (52 mg, 0.421 mmol), Na₂SO₄ (119 mg, 0.843 mmol) and TEA (78 µL, 0.562 mmol) in THF (2 mL) for 6 h, after work-up and purification on column chromatography (DCM/AcOEt, 7:3), gave nitrone QN16 as a yellow solid (26 mg, 26%); mp 152-4 °C; ¹H NMR (300 MHz, CDCl₃) δ 10.05 (s, 1H), 7.98 (s, 1H), 7.95 (d, *J=* 15.4 Hz, 1H), 7.86 (d, *J=* 9.2 Hz, 1H), 7.31 (dd, *J=* 9.2, 2.8 Hz, 1H), 7.07 (d, *J=* 15.4 Hz, 1H), 7.06 (d, *J=* 7.4 Hz, 1H), 4.24 (q, *J=* 7.1 Hz, 2H), 3.84 (s, 3H), 1.62 (s, 9H), 1.29 (t, *J=* 7.1 Hz, 3H); ¹³C NMR (75 MHz, CDCl₃) δ 167.1 (C), 159.0 (C), 148.5 (C), 144.1 (C), 139.5 (CH), 134.3 (CH), 131.2 (CH), 129.4 (C), 125.8 (CH), 124.8 (CH), 124.5 (CH), 123.7 (C), 106.1 (CH), 72.7 (C), 61.2 (CH₂), 56.0 (CH₃), 28.7 (3 x CH₃), 14.7 (CH₃). HRMS ESI_ACN. Calcd. for C₂₀H₂₄N₂O₄: 356.17361. Found: 356.17531. Anal. Calcd. for C₂₀H₂₄N₂O₄: C, 67.40; H, 6.79; N, 7.86. Found: C, 67.28; H, 6.82; N, 7.88.

### 2. Evaluation of Neuroprotection in an Experimental Model of Ischemia in Primary Neuronal Cultures

### 2.1. Materials and methods

Primary Neuronal Cultures. Primary neuronal cultures were prepared from rat embryos cerebral cortex following a procedure previously described by our group. Briefly, cell suspensions were prepared from cerebral cortices from E16 Sprague Dawley rat embryos and seeded on plastic multidishes precoated with 0.05 mg/mL poly-D-lysine at a density of 2.5 × 10⁵ cells/cm². Cells were kept at 37 °C in a 6.5% CO₂ atmosphere in high glucose DMEM medium supplemented with 15% heat-inactivated (56 °C, 30 min) fetal calf serum. After 24 h, culture medium was replaced by serum-free medium (DMEM/Ham's F12, 1:1 vol/vol, 5 mg/mL glucose, 2 mM L-glutamine, and 1 mM sodium pyruvate, supplemented with 100 µg/mL transferrin, 100 µM putrescine, 30 nM sodium selenite, 20 nM progesterone, and 5 µg/mL insulin). In these experiments, 6- to 7-days in vitro (DIV) neuronal cultures were used, containing 90% β-tubulin isotype III-positive mature neurons, as described previously.²³ All procedures associated with animal experiments were authorized by the Animal Experimentation Ethics Committee of the Hospital Universitario Ramón y Cajal (Madrid, Spain).

Experimental Ischemia in Neuronal Cultures and Treatments. Primary neuronal cultures were subjected to oxygen-glucose deprivation (OGD) to induce experimental ischemia. Cells cultured for 6- to 7- DIV were washed and placed in glucose-free DMEM medium (previously bubbled with 95% N₂/5% CO₂ for 30 min) and kept in a humidified anaerobic chamber containing a gas mixture of 95% N₂/5% CO₂ at 37 °C for 4 h (OGD 4h). Control group was placed in glucose-supplemented DMEM medium and kept in the normoxic incubator for 4h. After 4h of incubation under anoxic (OGD 4h) or normoxic (control) conditions, culture cells were quickly placed in serum-free normo-glycemic culture medium and let to reoxygenate, compounds or vehicle were added and cells were maintained in normoxic and normoglycemic conditions to recover for 24 h. Vehicle experimental group (R24h) included the same amount of vehicle solution than the groups tested with compounds (final concentration < 0.5% ethanol). The experimental procedure was blindly performed, assigning a random order to each group assayed. Compound assays were performed independently from four to eight times with different batches of cultures, and each experiment was run in quadruplicate.

Cell viability assays. To assess the potential neuroprotective effect of the compounds against OGD, cell viability was evaluated by quantification of living, metabolically active cells, as determined by the colorimetric assay using the photometric reduction of 3-(4,5-dimethylthiazol-2-yl)-2,5-diphenyl tetrazolium bromide (MTT) (Roche) to a blue formazan product. MTT assay was performed after the 24-hour-long recovery period -except for the OGD 4h group, for which MTT was added right after the end of the OGD period, with no recovery- by treating cells with 0.2 mg/mL MTT in the culture medium for 1.5 h at 37 °C in a 6.5% CO₂ atmosphere. After incubation, cells were lysed with an equal volume of lysis solution (10 mM HCl, 10% SDS) overnight. Values were quantified by absorbance at 595 nm (reference 690 nm) and expressed in Absorbance Units (AU). Decreased MTT activity denotes impairment of mitochondrial function and is indicative of cell damage.

Statistical Analysis. Data from each treatment were independently analyzed and their averaged values were used for statistical analysis. The treatment information was kept concealed throughout the study. Data were represented as mean ± SE. Analysis of variance (ANOVA) was performed to compare the data between multiple concentrations, following post hoc test when analysis of variance was significant. Statistical significance level was set at α = 0.05 using Prism statistical software (GraphPad Software).

### 2.2. Results

The compounds were tested in an experimental model of ischemia in order to assess their potential effect on cell viability preservation after an ischemic insult at several concentrations, from 0.1 to 250 µM.

As a consequence of the OGD period, a severe decrease in cell viability was observed, as measured in the unrecovered experimental group (OGD 4 h, 1.241 ± 0.03 AU, p < 0.0001 compared with control value, 1.916 ± 0.07 AU, Student's t test). This impairment in cell metabolism was partially reversed after recovery of normal oxygen and glucose levels for 24 h (R24h; 1.480 ± 0.02 AU, p < 0.0001 vs control, by Student's t test).

The addition of citicoline (100 µM) at the onset of the recovery period after OGD prompted a significant increase in cell viability (1.588 ± 0.02 AU, p < 0.01, by Student's t test) compared with R24h vehicle-treated group.

When standards NXY-059 or QN23 were added instead of citicoline, higher levels of cell viability were achieved (1.664 ± 0.07 AU and 1.695 ± 0.03 AU for 100 µM NXY-059 and 10 µM QN23, respectively).

According to the different structural pattern studied, exploration of O-propargyl motif at C6 position on ligands QN1 and QN2 rendered no remarkable overall protective effect in the concentration range tested.

When the compounds of the invention were tested at the different concentrations, the inventors surprisingly found that those bearing some aromaticity in 2 or 6 position showed a higher potency at doses of 1 uM, which are remarkably lower than those used for QN23 and NXY-059.

For example, a superior performance of the N-tert-Bu derivative in contrast to the N-tert-Bn substituted one was observed for the compounds QN4 and QN3, respectively, both substituted at C6 with the O-Bn group. In fact, QN4 showed a significant protective activity at 1 µM concentration (1.777 ± 0.04 AU).

The next group of QNs tested included different combinations over the quinoline core substituted with a methoxy group at C6. The simplest and the only one of the entire set carrying an electron-donating group at C2 (-OH, QN5) did not give a remarkable protective effect at the concentrations tested. Likewise, molecules QN6 and QN7 bearing a C(2)F substituent did not show significant differences to the value obtained for the vehicle-treated group (R24h).

The results of the substitution at C2 with other electron-withdrawing groups, different from Cl as in QN23, such as an arylsulfone (QN8-11), a substituted phenyl group (QN12-15) or a α,β-unsaturated carboxylic ester (QN16) showed higher effect than vehicle R24h at 1 µM dose. The *p*-NO₂ substituted QN14 and QN15, in were safe and potent. Remarkably, QN14 showed interesting cell viability results, significantly higher than vehicle-treated group (1.742 ± 0.04 AU at 1 µM).

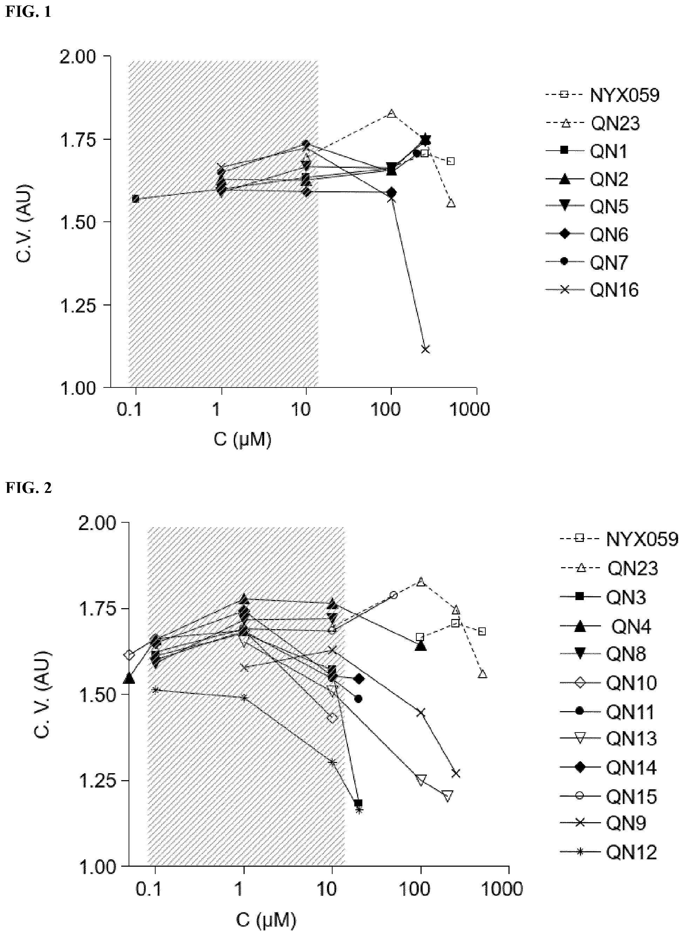

The whole data are provided in figures 1 and 2.

With these results, in order to express cell viability as a more useful concept in the search for protective compounds in the ischemic disease, neuroprotection activity was defined as the effect that achieved a cellular viability higher than the vehicle-treated group (R24h), which set the basal neuroprotection value (0%). Cell viability observed in the control group was set as 100% of neuroprotection. Consequently, the neuroprotection values obtained for standards NXY-059, QN23, and some of the compounds have been gathered together in Table 1 below:

**Table 1**

| Compound | Conc (µM) | Neuroprotection (%) |
|---|---|---|
| NXY-059⁽¹⁾ | 100 | 41.93 ± 2.26 |
| QN23⁽¹⁾ | **10** | 49.00 ± 0.89 |
| QN1⁽¹⁾ | 1 | 26.89 ± 0.88 |
| | 10 | 34.81 ± 0.78 |
| | 100 | 41.26 ± 1.91 |
| QN2⁽¹⁾ | 1 | 33.66 ± 0.73 |
| | 10 | 33.26 ± 0.93 |
| | 100 | 39.98 ± 0.77 |
| **QN3⁽²⁾** | **1** | **45.23 ± 1.00** |
| **QN4⁽²⁾** | **1** | **67.89 ± 1.44** |
| QN5⁽¹⁾ | 1 | 24.50 ± 0.54 |
| QN6⁽¹⁾ | 1 | 26.27 ± 0.89 |
| | 10 | 25.08 ± 1.03 |
| QN7⁽¹⁾ | 1 | 38.45 ± 0.62 |
| **QN8⁽²⁾** | **1** | **54.11** ± **2.27** |
| **QN10⁽²⁾** | **1** | **47.25** ± **0.94** |
| **QN11⁽²⁾** | **1** | **46.50** ± **1.94** |
| **QN13⁽²⁾** | **1** | **39.29** ± **1.16** |
| **QN14⁽²⁾** | **1** | **60.10 ± 1.24** |
| **QN15⁽²⁾** | **1** | **48.09 ± 1.21** |

| | | |
|---|---|---|
| (1): COMPOUND FOR COMPARATIVE PURPOSE; (2): COMPOUND OF THE INVENTION | | |

All the compounds of the invention ((2)) were remarkably more potent than the references NXY-059 and QN23. Not only that, but also, they were more efficient at remarkably lower doses than NXY-059.

Furthermore, QN4, QN8, QN14 and QN15 were found to provide the highest neuroprotection effect using a remarkably lower doses than the one of QN23: 1 µM for the compounds of the invention vs 10 µM for QN23, meaning increased pharmacological potency.

In terms of neuroprotection capacity, the effect induced by the compounds of the invention is still higher than reference compound NXY-059, the furthest-reaching -but failed- chemical compound in clinical development for the treatment of CI so far.

Remarkably, the compounds of the invention are non-toxic and safe compounds at all tested doses, despite the toxicological alert described for the nitro group. And were soluble in an acceptable range of concentration.

## Claims

1. A compound of formula (I) or a pharmaceutically salt or hydrate thereof: 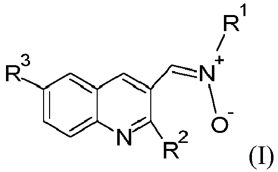 wherein:
R¹ represents (C₁-C₅)alkyl or an aromatic ring system;
R² represents halogen, a known aromatic ring system or -S(O)₂R⁴,
R³ represents -O-(CH₂)ₙ-R₅;
R⁴ represents a known aromatic ring system;
R⁵ represents (C₁-C₅)alkyl, (C₁-C₅)alkenyl, (C₁-C₅)alkynyl, (C₁-C₅)haloalkyl, or a known aromatic ring system;
n is an integer value selected from 0 and 1;
the term "known aromatic ring system" means an aromatic ring having 5 or 6 members selected from: -CH-, -CR⁶-, -O-, -N-, and -S-;
R⁶ represents halogen, (C₁-C₁₀)alkyl, (C₂-C₁₀)alkenyl, (C₂-C₁₀)alkynyl, (C₁-C₁₀)haloalkyl, (C₂-C₁₀)haloalkenyl, (C₂-C₁₀)haloalkynyl, -O-(C₁-C₁₀)alkyl, -CF₃, -NO₂, -OH, -CN, -COOR₇, - R⁸C(O)OR₉, or -C(O)R¹⁰; and
R⁷ to R¹⁰ represents (C₁-C₁₀)alkyl, (C₂-C₁₀)alkenyl, (C₂-C₁₀)alkynyl, or (C₁-C₁₀)haloalkyl;
provided that:
when R² represents halogen, then R³ represents -O-(CH₂)ₙ-R⁵, being R⁵ an aromatic ring system; and
when R³ represents -O-(CH₂)ₙ-R⁵, being R⁵ selected from the group consisting of (C₁-Cs)alkenyl, (C₁-C₅)alkynyl, and (C₁-C₅)haloalkyl, then R² represents an aromatic ring system or -S(O)₂R⁴.

2. The compound of formula (I) according to claim 1, wherein R² represents halogen or a known aromatic ring system.

3. The compound of formula (I) according to claim 1, wherein R⁵ is selected from (C₁-C₅)alkyl and an aromatic ring system.

4. The compound of formula (I) according to any of the preceding claims, wherein the known aromatic ring system has 6 members.

5. The compound of formula (I) according to any of the preceding claims, wherein the aromatic ring system has 6 members selected from -CH- and -CR⁶-, R⁶ being as defined in claim 1.

6. The compound of formula (I) according to claim 5, wherein R⁶ is selected from halogen, (C₁-C₅)haloalkyl, and -NO₂.

7. The compound of formula (I) according to any of the preceding claims, wherein R¹ represents (C₁-C₅)alkyl or a benzyl.

8. The compound of formula (I) according to any of the preceding claims, wherein R² represents halogen or a phenyl group optionally substituted with one or more groups selected from (C₁-Cs)haloalkyl, and -NO₂.

9. The compound of formula (I) according to any of the preceding claims, wherein R³ represents -O-(C₁-C₅)alkyl or a benzyloxy group.

10. The compound of formula (I) according to any of the preceding claims, which is selected from the group consisting of:
(Z)-N-Benzyl-1-(6-(benzyloxy)-2-chloroquinolin-3-yl)methanimine oxide (QN3);
(Z)-N-tert-Butyl-1-(6-(benzyloxy)-2-chloroquinolin-3-yl)methanimine oxide (QN4);
(Z)-N-Benzyl-1-(6-methoxy-2-(phenylsulfonyl)quinolin-3-yl)methanimine oxide (QN8);
(Z)-N-tert-Butyl-1-(6-methoxy-2-(phenylsulfonyl)quinolin-3-yl)methanimine oxide (QN9);
(Z)-N-Benzyl-1-(2-((4-nitrophenyl)sulfonyl)-6-methoxyquinolin-3-yl)methanimine oxide (QN10);
(Z)-N-tert-Butyl-1-(2-((4-nitrophenyl)sulfonyl)-6-methoxyquinolin-3-yl)methanimine oxide (QN11);
(Z)-N-Benzyl-1-(6-methoxy-2-(4-(trifluoromethyl)phenyl)quinolin-3-yl)methanimine oxide (QN12);
(Z)-N-tert-butyl-1-(6-methoxy-2-(4-(trifluoromethyl)phenyl)quinolin-3-yl)methanimine oxide (QN13);
(*Z*)-*N*-Benzyl-1-(6-methoxy-2-(4-nitrophenyl)quinolin-3-yl)methanimine oxide (QN14); and
(*Z*)-*N*-*tert*-Butyl-1-(6-methoxy-2-(4-nitrophenyl)quinolin-3-yl)methanimine oxide (QN15).

11. The compound of formula (I) as defined in any of the claims 1-9, which is selected from (Z)-N-tert-butyl-1-(6-(benzyloxy)-2-chloroquinolin-3-yl)methanimine oxide (QN4), (*Z*)-*N*-Benzyl-1-(6-methoxy-2-(4-nitrophenyl)quinolin-3-yl)methanimine oxide (QN14) and (*Z*)-*N*-*tert*-Butyl-1-(6-methoxy-2-(4-nitrophenyl)quinolin-3-yl)methanimine oxide (QN15).

12. A pharmaceutical composition comprising a therapeutically effective amount of the compound of formula (I) as defined in any of the preceding claims 1-10, together with one or more pharmacologically acceptable excipients or carriers.

13. A compound of formula (I) as defined in any of the preceding claims 1-10 for use in therapy.

14. A compound of formula (I) as defined in any of the preceding claims 1-10 for use as neuroprotective agent.

15. A compound of formula (I) as defined in any of the preceding claims 1-10 for use in the treatment or prevention of cerebral ischaemia or stroke.
